# EUROPÄISCHE PATENTSCHRIFT

(11) **EP 0 446 649 B1**
(45) Veröffentlichungstag und Bekanntmachung des Hinweises auf die Patenterteilung: **10.11.1993**
(21) Anmeldenummer: 91102218.4
(22) Anmeldetag: 16.02.1991
(51) Int. Cl.: C07C 303/42

(54) **Verfahren zur Herstellung von Na-Salz der Diethylmetanilsäure**
Process for the preparation of sodium 3-diethylaminobenzene-sulfonate
Procédé pour la préparation de 3-diéthylaminobenzènesulfonate de sodium

(30) Priorität: 28.02.1990 DE 4006225
(43) Veröffentlichungstag der Anmeldung: 18.09.1991
(73) Patentinhaber: BASF Aktiengesellschaft, 67063 Ludwigshafen (DE)
(72) Erfinder: Kolb, Albrecht, Dr., W-6719 Weisenheim (DE); Guenther, Rolf, W-6800 Mannheim 31 (DE); Apel, Wolfram, W-7529 Forst (DE); Rosinger, Manfred, W-6737 Boehl-Iggelheim (DE); Munzinger, Manfred, W-6716 Dirmstein (DE)

(56) Entgegenhaltungen:
- EP-A- 0 293 744
- US-A- 2 488 885
- CHEMICAL ABSTRACTS, Band 34, 1940, Spalte 3246, Zusammenfassung Nr. 4,5, Columbus, Ohio, US; V.A. IZMAIL'SKII et al.: "Alkylation. VII. Preparation of diethylmetanilic acid and diethyl-m-aminophenol"

## Beschreibung

Die Erfindung betrifft ein Verfahren zur Herstellung von Na-Salz der Diethylmetanilsäure (Wertprodukt) durch Reaktion von Methanilsäure, Ethylchlorid und Natronlauge und Abtrennung des bei der Reaktion entstehenden NaCl aus dem Reaktionsprodukt mittels eines Dekanters.

Die Reaktion verläuft nach folgender Gleichung und angegebenen Reaktionsbedingungen.

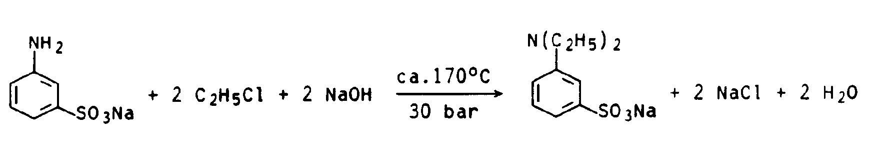

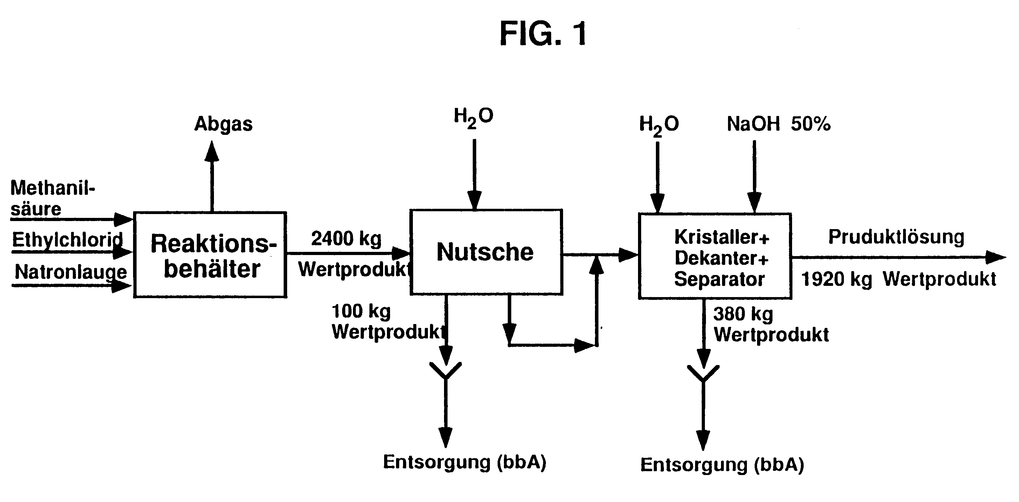

Die Herstellung erfolgt nach dem Stand der Technik wie nachstehend, wobei in einem vereinfachten Verfahrensschema, siehe Figur 1, das Verfahren und die Mengenbilanz bzgl. des Na-Salzes der Diethylmetanilsäure - im weiteren Wertprodukt genannt - aufgezeigt sind.

Während der Reaktion im Reaktionsbehälter - wobei ca. 2400 kg/Charge Wertprodukt entstehen - kristallisiert ein Teil des NaCl aus, dieses wird nach der Reaktion auf einer Nutsche abfiltriert und anschließend mit Wasser gewaschen. Die Waschlösung, die überwiegend Wertprodukt und in geringen Mengen NaCl beinhaltet, wird in die Produktlösung zurückgeleitet, und das NaCl und ca. 100 kg Wertprodukt, nach der Waschung in Wasser gelöst, werden der Entsorgung zugeführt. Die Produktlösung wird vor der anschließenden Kristallisation in einem Kristallisationsbehälter mit Wasser bei 80°C auf 21,5° Be' eingestellt. Nach Zugabe von NaOH-50 %ig zur Reduzierung der Wertprodukt-Löslichkeit wird die Produktlösung unter Rühren auf 20-35°C abgekühlt, dabei kristallisiert das Wertprodukt aus. In nachfolgenden Verfahrensschritten wird mittels eines Dekanters und eines Separators das Kristallisat abgetrennt, hierbei wird als Endprodukt die Produktlösung als Dekanter- und Separatoraustrag mit einem Gehalt von ca. 1920 kg Wertprodukt gewonnen und das Separatorfiltrat mit ca. 380 kg Wertprodukt der Entsorgung zugeführt.

Als Nachteil des Verfahrens erweist sich - wie aus der Mengenbilanz ersichtlich - daß ca. 480 kg Wertprodukt = ca. 20 % der Entsorgung zugeführt werden und damit ein hoher Prozentsatz an Wertprodukt verlorengeht.

Es stellte sich daher die Aufgabe ein Verfahren zur Herstellung des Na-Salzes der Diethylmetanilsäure zu konzipieren, wobei obiger Verlust des Wertproduktes weitgehend vermieden wird.

Diese Aufgabe wird erfindungsgemäß dadurch gelöst, daß das vom kristallinen NaCl befreite Reaktionsprodukt in einem Phasentrenngefäß NaOH-50 %ig in einem solchen Verhältnis zugegeben wird, daß die Phasentrennung derart erfolgt, daß die organische Phase ca. 99,5 % des Wertprodukts und die wäßrige Phase ca. 0,5 % des Wertproduktes enthält.

Ein Ausführungsbeispiel des erfindungsgemäßen Verfahrens mit den wesentlichen Merkmalen ist in der Zeichnung dargestellt und wird im folgenden näher beschrieben.

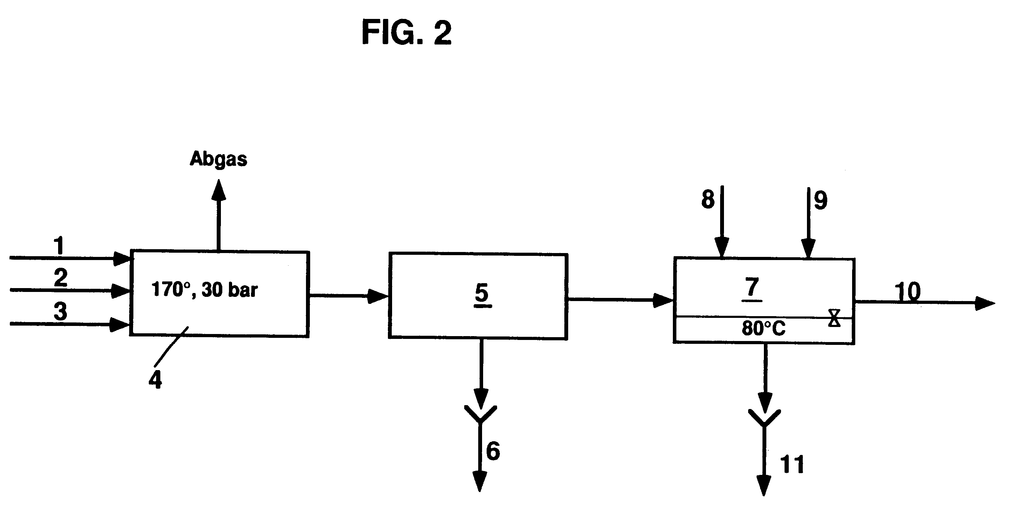

Die Zeichnung - Figur 2 - zeigt ein vereinfachtes Verfahrensschema mit Mengenbilanz und Reaktionsbedingungen.

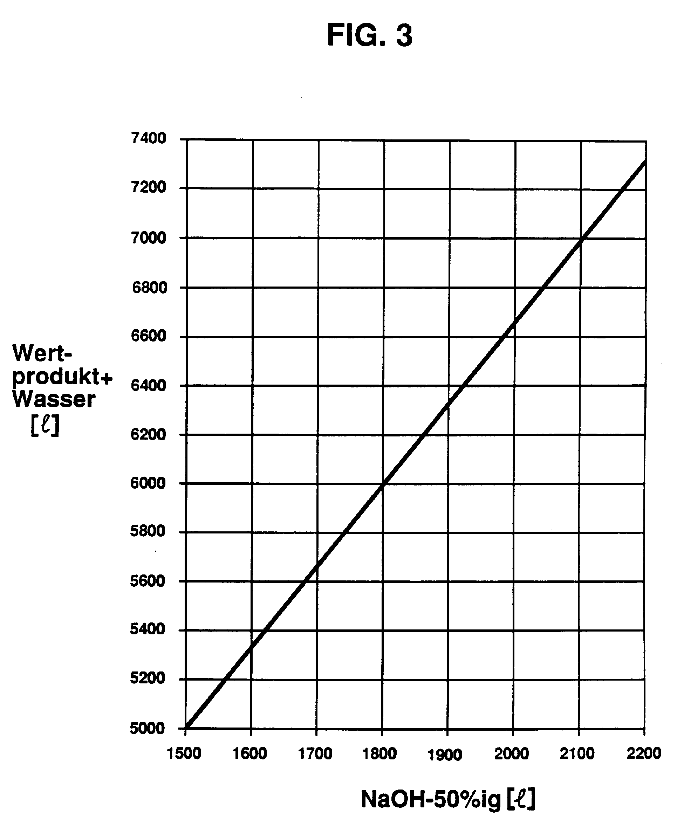

Nach der Reaktion der Reaktanden - Methanilsäure 1, Ethylchlorid 2 und NaOH 3 - wobei 2400 kg Wertprodukt entstehen, gemäß vorangehender Reaktionsgleichung und vorangehenden Reaktionsbedingungen, in einem Rührbehälter 4, erfolgt in einem Dekanter 5 mit 430 mm Durchmesser die Abtrennung von 1000 kg NaCl 6 aus der Produktlösung bei gleichzeitiger Abtrennung von ca. 9 kg Wertprodukt. Das Reaktionsprodukt wird mittels Druck durch ein Tauchrohr aus dem Rührbehälter herausgefördert und mittels einer Pumpe dem Dekanter zugeführt. In einem anschließenden Phasentrenngefäß - einem Rührbehälter mit Heizmantel - wird die aus dem Dekanter zuströmende Produktlösung mittels Wasser 8 auf 21,5° Be' eingestellt und mittels Heizung auf ca. 80°C gehalten. Die Phasentrennung stellt sich nach Zugabe - unter Rühren - von NaOH-50 %ig 9 und anschließender ca. 1stündiger Phasentrennzeit ein. Die Trennung in organische Phase 10 und wäßrige Phase 11 im Verhältnis 1:1 erfordert eine Zugabe der NaOH-50 %ig gemäß Diagramm, siehe Figur 3. Die wäßrige Phase - Spuren von NaCl und ca. 1 kg Wertprodukt enthaltend - wird als behandlungsbedürftiges Abwasser zur Entsorgung geleitet, die organische Phase, 2390 kg Wertprodukt enthaltend, wird zur Weiterverarbeitung geleitet.

Der Verlust an Wertprodukt gemäß dem erfindungsgemäßen Verfahren beträgt ca. 10 kg = 0,4 % gegenüber ca. 480 kg = 20 % nach dem Verfahren des Standes der Technik.

## Patentansprüche

1. Verfahren zur Herstellung von Na-Salz der Diethylmetanilsäure (Wertprodukt) durch Reaktion von Methanilsäure, Ethylchlorid und Natronlauge und Abtrennung des bei der Reaktion entstehenden NaCl aus dem Reaktionsprodukt mittels eines Dekanters, dadurch gekennzeichnet, daß das vom kristallinen NaCl befreite Reaktionsprodukt in einem Phasentrenngefäß NaOH-50 %ig in einem solchen Verhältnis zugegeben wird, daß die Phasentrennung derart erfolgt, daß die organische Phase ca. 99,5 % des Wertprodukts und die wäßrige Phase ca. 0,5 % des Wertproduktes enthält.

## Claims

1. A process for preparing sodium 3-ethylaminobenzenesulfonate by reacting metanilic acid, ethyl chloride and sodium hydroxide and removing the resulting NaCl from the reaction product by means of a decanter, characterized in that the reaction product freed from crystalline NaCl is admixed in a phase separation vessel with 50% strength NaOH in such a ratio that phase separation takes place in such a way that the organic phase ends up with about 99.5% of the desired product and the aqueous phase with about 0.5% of the desired product.

## Revendications

1. Procédé pour la préparation du sel de Na de l'acide diéthylmétanilique (produit de valeur) par réaction de l'acide métanilique, du chlorure d'éthyle et de la lessive de soude et séparation, à partir du produit de réaction, du NaCI formé à l'aide d'un décanteur, caractérisé en ce que l'on ajoute au produit de réaction débarrassé du NaCI cristallisé, dans un récipient à séparation de phases, NaOH à 50 % en proportions telles que la séparation de phases se produise en une phase organique qui contient environ 99,5 % du produit de valeur et une phase aqueuse qui en contient environ 0,5 %.
